# EUROPÄISCHE PATENTSCHRIFT

(11) **EP 1 074 272 B1**
(45) Veröffentlichungstag und Bekanntmachung des Hinweises auf die Patenterteilung: **29.09.2004**
(21) Anmeldenummer: 00116538.0
(22) Anmeldetag: 31.07.2000
(51) Int. Cl.: A61M 1/36, B01L 3/14

(54) **Adapterstopfen für ein Aufnahmegefäss für Körperflüssigkeiten**
Plug for a recipient for body liquids
Bouchon pour un récipient à liquides corporels

(30) Priorität: 02.08.1999 DE 19936295
(43) Veröffentlichungstag der Anmeldung: 07.02.2001
(73) Patentinhaber: Müller, Hans, 81547 München (DE)
(72) Erfinder: Müller, Hans, 81547 München (DE)
(74) Vertreter: Fleuchaus, Leo, Dipl.-Ing.

(56) Entgegenhaltungen:
- EP-A- 0 240 144
- DE-A- 3 210 730
- DE-A- 19 833 435
- US-A- 4 568 345
- US-A- 5 425 465

## Beschreibung

Die Erfindung betrifft einen Adapterstopfen zum Be- und Entladen eines Auf nahmegefäßes für Körperflüssigkeiten, welches für die Bestrahlung besagter Körperflüssigkeiten geeignet ist.

Zu medizinischen Therapieverfahren werden dem Patienten entnommene Körperflüssigkeiten mit UV Licht bestrahlt. Vorrichtungen zu diesem Zweck kennt man aus der Deutschen Patentschrift 195 31 751, wobei die entnommene Körperflüssigkeit in ein Aufnahmegefäß, z.B. eine Quarzküvette, überführt und anschließend mit UV Licht bestrahlt wird. Zur Be- und Entladung wird die Körperflüssigkeit mit einer Spritze durch eine Be- und Entladeöffnung eines Adapterstopfens, der die Quarzküvette verschließt, gespritzt. Der hierbei in der Quarzküvette entstehende Überdruck kann durch eine Lüftungsöffnung am gegenüberliegenden Stopfen oder auch direkt am Adapterstopfen (Deutsche Patentanmeldung 198 33 435.4) entweichen. Bei vorschriftsmäßiger Benutzung, wobei die Quarzküvette beim Einspritzen der Körperflüssigkeit in das Aufnahmegefäß so gehalten werden soll, daß die Lüftungsöffnung nach oben zeigt, sollte ein ungehinderter Gasaustausch stattfinden ohne daß dabei Körperflüssigkeit durch die Lüftungsbohrung entweicht.

Es hat sich jedoch gezeigt, daß es trotz sachgemäßer Handhabung zu einem Austreten von Körperflüssigkeit durch die Lüftungsbohrung am Adapterstopfen kommen kann. Besonders durch ein zu schnelles Einspritzen der Körperflüssigkeit durch den Adapterstopfen kommt es kurzzeitig zu einer relativ großen Mengen an Körperflüssigkeit in dem Einspritzkanal, die diesen vollständig ausfüllt. Unter solchen Bedingungen führt der Druck mit dem die Flüssigkeit dabei eingespritzt wird dazu, daß geringe Mengen an Körperflüssigkeit in die Lüftungsbohrung, die neben der Be- und Entladeöffnung angebracht ist, gelangen oder durch sie hindurch austreten. Ein Austreten von Körperflüssigkeit kann auch auf einen strömungsbedingten Rückfluß oder ein Aufschäumen der Körperflüssigkeit zurückzuführen sein. Als Folge davon führt die ausgetretene Körperflüssigkeit zu Verschmutzungen der Außenflächen des Adapterstopfens bzw. der Quarzküvette. Durch das Einführen einer außen verschmutzten Quarzküvette in das Bestrahlungsgerät kann auch dieses verschmutzt werden. Solche Verschmutzungen mit Körperflüssigkeiten stellen bevorzugte Keimreservoirs dar und führen damit zu einem Kontaminationsrisiko. Es kommt hinzu, daß beim Entnehmen der Körperflüssigkeit durch Absaugen aus der Quarzküvette, die in die Lüftungsöffnung gelangte Körperflüssigkeit mit abgesaugt wird. Da davon ausgegangen werden kann, daß die Außenbereiche nicht steril sind, wird hierbei die gesamte Probe kontaminiert.

Durch die US 5425465 sind Ventilstopfen für ein Aufnahmegefäß für Körperflüssigkeiten bekannt, welche mit Entenschnabelventilen oder ähnlich wirkenden Ventilen versehen sind. Bei einem der Ventilstopfen ist im auf dem Rand des Aufnahmegefäßes aufliegenden Flansch innerhalb des Randes ein Einweg-Entlüftungsventil vorgesehen, das sich beim Absaugen der Körperflüssigkeit aus dem Aufnahmegefäß öffnet.

Auch in der EP 240 144 A1 ist ein Adapterstopfen mit einer durch ein Bakterienfilter verschließbaren Entlüftungleitung versehen, welche in die Einspritzkammer mündet. Die Erfahrung lehrt, daß derartige Bakterienfilter keinen sicheren Schutz gegen ein Kontaminationsrisiko darstellen.

Es ist daher Aufgabe der Erfindung eine Vorrichtung zur sterilen Beladung und Entladung einer Quarzküvette zur Bestrahlung von Körperflüssigkeit in einem Bestrahlungsgerät zur Verfügung zu stellen, bei welcher gewährleistet ist, daß trotz unsachgemäßer Handhabung keine Kontamination der Außenflächen des Aufnahmegefäßes bzw. der Bestrahlungsvorrichtung erfolgt, wobei aus Kostengründen sowie um eine möglichst universelle Verwendbarkeit zu gewährleisten, eine Lösung gesucht wird, die einen möglichst geringen apparativen Aufwand erfordert.

Diese Aufgabe wird erfindungsgemäß durch einen Adapterstopfen für ein Aufnahmegefäß für Körperflüssigkeiten gemäß Anspruch 1 gelöst.

Beim Einfüllen der Körperflüssigkeit in die Küvette durch den Adapterstopfen verhindert die erfindungsgemäße Trennwand das unerwünschte Austreten von Spritzern der Körperflüssigkeit durch die Lüftungsbohrung, indem der Flüssigkeitsstrom direkt zur aufnahmegefäßseitige Öffnung gelenkt wird, ohne in direkten Kontakt mit der Lüftungsbohrung, die in Lüftungskammer mündet, zu kommen. Da die Lüftungsbohrung am oberen Rand der Steckhülse angebracht ist, muß zum Be- und Entladen des Aufnahmegefäßes der Adapterstopfen nur geringfügig aus dem Aufnahmegefäß herausgezogen werden, sodaß die Lüftungsöffnung am oberen Rand der Steckhülse freiliegt. Um ein zu weites Herausziehen des Steckstopfens und damit eine instabile Handhabung zu vermeiden, ist es vorteilhaft die Lüftungsbohrung nahe dem oberen Rand anzubringen. Durch diese Lüftungsbohrung kann nun - im herausgezogenen Zustand - beim Einspritzen der Körperflüssigkeit Luft aus dem Aufnahmegefäß ausströmen und so den entstehenden Überdruck ausgleichen. Es kann vorteilhaft sein den Adapterstopfen über eine oder mehrere, zusätzlich an der Außenseite der Steckhülse angebrachte Lippen im Aufnahmegefäß zu fixiern, um dem entstehenden geringen Ausdehnungsdruck beim Beladen standzuhalten. Nach erfolgter Beladung wird die Steckhülse des Adapterstopfens ganz auf die Küvette aufgeschoben. Die Lüftungsöffnung liegt nun an der Innenwand des Aufnahmegefäßes an und ist damit dicht verschlossen. Aus einem derart abgeschlossenen System kann während der Bestrahlung oder weiteren Handhabung des Aufnahmegefäßes keine Körperflüssigkeit entweichen, so daß eine Kontamination der Bestrahlungsvorrichtung ausgeschlossen ist. Nach erfolgter Bestrahlung wird die Steckhülse wieder etwas aus der Küvette herausgezogen, damit die Lüftungsbohrung erneut frei liegt und einen Gasaustausch ermöglicht. Zu Beginn einer Entnahme der Körperflüssigkeit, wird das Aufnahmegefäß zunächst leicht nach unten oder waagrecht gehalten, während dem Absaugen der Körperflüssigkeit wird es dann langsam in eine senkrechte Position gebracht. Durch die sich langsam verändernde Schräglage läuft alle Körperflüssigkeit an der schräg gehaltenen Wand des Aufnahmegefäßes zusammen und kann so vollständig abgesaugt werden.

Der herausragende Vorteil des erfindungsgemäßen Adapterstopfens ist, daß durch die Anordnung der Lüftungsbohrung und der Trennwand im Adapterstopfen einerseits die Be- und Entlüftung während des Be- und Entladevorganges des Aufnahmegefäßes mit Körperflüssigkeit gewährleistet ist und zum anderen während der Bestrahlung das Aufnahmegefäß dicht verschlossen ist. Selbst bei ungeschickter Handhabung ist so gewährleistet, daß keine Kontamination an der Außenseite des Aufnahmegefäßes und infolgedessen der Bestrahlungsvorrichtung auftritt. Da es sich bei diesem Adapterstopfen, wie auch dem ganzen Aufnahmegefäß, um eine Einwegvorrichtung handelt, die steril verpackt ist, kann man davon auszugehen, daß eine Kontamination sicher verhindert wird. Ebenso ist eine Kontamination der Bestrahlungsvorrichtung selbst durch mehrfache unsachgemäße Handhabung ausgeschlossen.

Gemäß einer Ausführungsform der Erfindung verläuft die Trennwand über die gesamte Innenlänge der Steckhülse. Die eingespritzte Körperflüssigkeit wird folglich entlang der Trennwand bis in das Aufnahmegefäß gelenkt. Ein Gasaustausch und damit die Belüftung des Aufnahmegefäßes erfolgt über den Luftraum im Aufnahmegefäß. Aufgrund des Abstandes der Lüftungsbohrung vom einlaufenden Flüssigkeitsstrom ist bei sachgemäßer Handhabung das Verschmutzen der Lüftungsöffnung und damit ein Austreten von Körperflüssigkeit nahezu unmöglich.

Der Innenraum der Lüftungskammer reicht gemäß einer Ausführungsform gleich weit in den Stopfen hinein wie der Innenraum der Einspritzkammer hinein reicht, der mit der Be- und Entladeöffnung verbunden ist. Dieser Aufbau ermöglicht eine optimale Ausnutzung der Plazierungsvarianten für die Lüftungsbohrung. Vorzugsweise wird die Lüftungbohrung am hinteren Ende der Steckhülse angebracht, um eine Be-/Entlüftung bereits bei geringem Herausziehen des Stopfens zu ermöglichen.

Eine besondere Ausführungsform der Erfindung sieht vor, daß der Abstand der Lüftungsbohrung vom freien Ende der Steckhülse größer ist, als der Abstand von der vordere Stirnseite der Trennwand zum freien Ende der Steckhülse. Bei einer derart kürzeren Trennwand ist gewährleistet, daß der kontinuierliche Flüssigkeitsstrom direkt zur aufnahmegefäßseitigen Öffnung der Einspritzkammer gelenkt wird, gleichzeitig ist der Gasaustausch über die zweite Kammer besonders erleichtert, da die beiden Kammern über einen gemeinsamen Luftraum am Ende der Steckhülse verbunden sind.

Weiterhin ist vorteilhafterweise der Innenraum der Lüftungkammer kürzer als der Innenraum der Einspritzkammer. Dadurch ist die Gesamtstabilität des Adapterstopfens erhöht.

Im Folgenden wird die Erfindung anhand einer Zeichnung von Ausführungsbeispielen näher erläutert. Es zeigen:
- Fig. 1: einen Längsschnitt durch einen Adapterstopfen für ein Aufnahmegefäß zur Bestrahlung von Körperflüssigkeiten;
- Fig. 2: eine Draufsicht auf den Adapterstopfengemäß Fig 1;
- Fig. 3: einen Längsschnitt durch einen Adapterstopfen mit kurzer Trennwand und ein Aufnahmegefäß für Körperflüssigkeiten zum Bestrahlen in einem Bestrahlungsgerät, wobei das Aufnahmegefäß mit einen Adapterstopfen verschlossen ist;
- Fig. 3a: einen Längsschnitt durch einen Adapterstopfen und ein Aufnahmegefäß für Körperflüssigkeiten gemäß Fig 3, wobei der Adapterstopfen in der Be- und Entlüftungsposition ist.
- Fig. 4: einen Längsschnitt durch einen Adapterstopfen mit kurzer Lüftungskammer und langer Trennwand

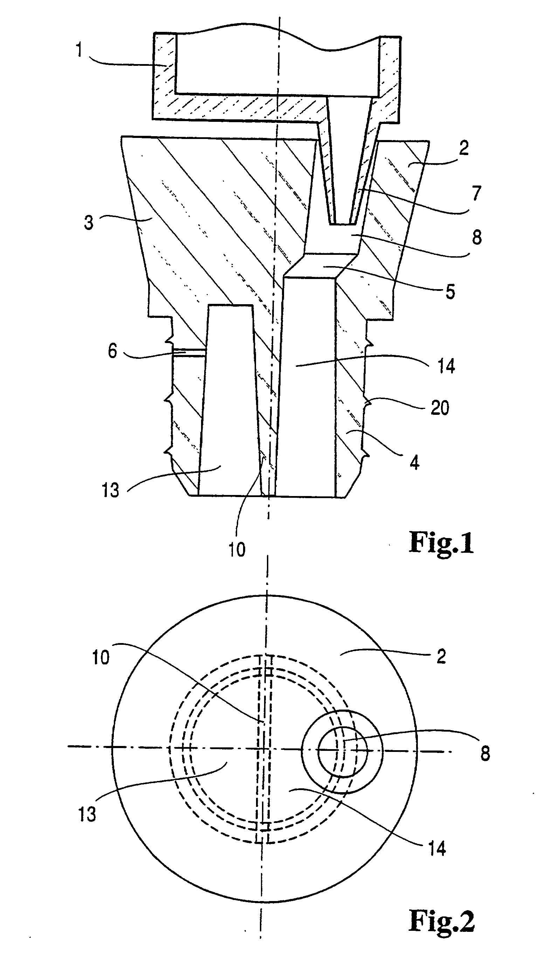

**Fig. 1** zeigt einen Adapterstopfen **2**, der einstückig eine Steckhülse **4** und einen trichterförmigen Aufsteckkonus **3** umfaßt. In diesen Aufsteckkonus kann eine Spritze **1** eingesetzt werden. Dabei ragt der Nadelkonus **7** der Spritze in eine die Spritze stabilisierende Durchbohrung **8** des Adaperstopfens, welche weiter in die Durchbohrung zum Be- und Entladen des Aufnahmegefäßes **5** sowie die Einspritzkammer **14** mündet. Die Einspritzkammer **14** ist im Innenbereich des Adapterstopfens durch eine Trennwand **10** von einer zweiten Kammer, der Lüftungskammer **13** abgetrennt, in die die Lüftungsbohrung **6** mündet. Die Position der Trennwand ist vorzugsweise mittig. Die Lüftungsbohrung **6** ist in der Lüftungskammer **13** und im hinteren Bereich der Steckhülse **4** angebracht. Durch diese Lüftungsbohrung **6** findet ein Luftaustausch beim Be- und Entladen eines Aufnahmegefäßes statt. Optional sind an der Außenseite der Steckhülse **4** radial verlaufende Lippen **20** angebracht, durch die der Adapterstopfen in dem Aufnahmegefäß entgegen den beim Belanden entstehenden Ausdehnungsdruck fixiert wird.

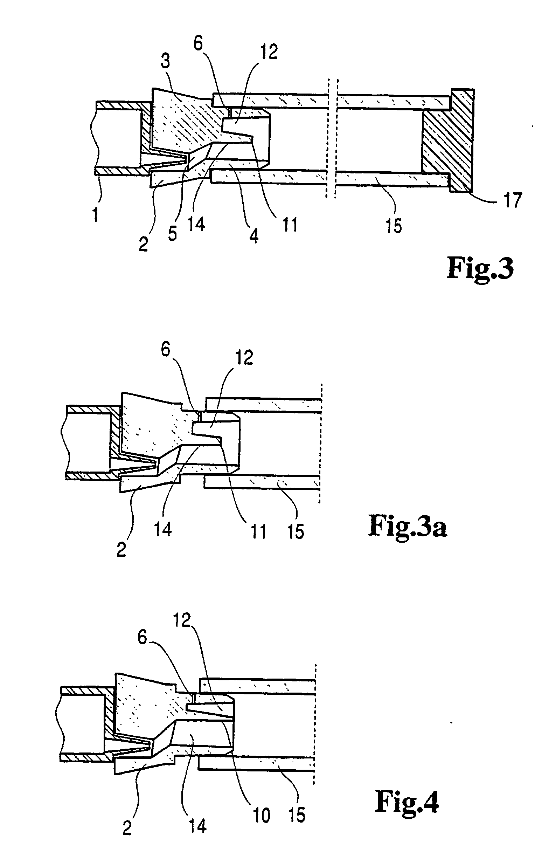

In **Fig. 2** ist eine Draufsicht auf den in **Fig. 1** abgebildeten Adapterstopfens gezeigt.

**Fig. 3** stellt den verschlossenen Zustand eines bekannten Aufnahmegefäßes **15** zur Bestrahlung von Körperflüssigkeiten dar. Dabei ist der Adapterstopfen **2,** in der erfindungsgemäßen Verwendung, dicht schließend in das Aufnahmegefäß 15 eingesteckt. Der dargestellte Adapterstopfen unterscheidet sich von den in Fig. 1 dargestellten, durch eine verkürzte Lüftungskammer **12**, sowie eine kürzere Trennwand **11**. Die Lüftungsbohrung **6** ist durch die außen anliegende Wand des Aufnahmegefäßes abgedichtet. Am hinteren Ende verschließt zusätzlich ein weiteres Verschlußelement **17** das Aufnahmegefäß **15**.

In **Fig. 3a** ist der Adapterstopfen **2** gemäß Fig. 3 in einer Position dargestellt, die zur Be- und Entladung des Aufnahmegefäßes geeignet ist. Die Lüftungsöffnung **6** befindet sich nun außerhalb des Aufnahmegefäßes, so daß ein ungehinderter Gasaustausch beim Be- und Entladen des Aufnahmegefäßes mit Körperflüssigkeiten stattfinden kann.

**Fig. 4** stellt eine weitere Ausführungsform des Adapterstopfens dar. Im Vergleich zu Fig. 1 ist die Lüftungskammer **12** verkürzt und die Trennwand **10** nicht mittig.

## Patentansprüche

1. Adapterstopfen für ein Aufnahmegefäß für Körperflüssigkeiten welches einem Aufsteckkonus (3) zur Aufnahme eines Nadelkonus (7) einer Snritze (1) sowie eine in eine aufnahmegefäßseitige Steckhülse (4) des Adapterstopfens mündende Lüftungsbohrung (6) aufweist, wobei
in der Steckhülse (4) eine Trennwand (10; 11) angebracht ist, welche den Innenraum der Steckhülse in eine Lüftungskammer (12; 13) und eine Einspritzkammer (14) unterteilt, und daß die Be- und Entladeöffnung (5) in die Einspritzkammer (14) und die Lüftungsbohrung (6) in die Lüftungskammer (12; 13) mündet, **dadurch gekennzeichnet, daß** die Lüftungsbohrung in einem hinteren Bereich der Steckhülse so angebracht ist, daß sie bei vollständig in das Aufnahmegefäß eingesteckter Steckhülse durch die Innenwand des Aufnahmegefäßes verschlossen wird.

2. Adapterstopfen gemäß Anspruch 1
**dadurch gekennzeichnet,**
**daß** der Abstand der Lüftungsbohrung (6) vom freien Ende der Steckhülse (4) größer als der des vorderen Stirnseite der Trennwand (11) vom freien Ende der Steckhülse (4) ist.

3. Adapterstopfen gemäß Anspruch 1 oder 2
**dadurch gekennzeichnet,**
**daß** die Trennwand (10) über die gesamte Innenlänge der Steckhülse (4) verläuft.

4. Adapterstopfen gemäß einem der Ansprüche 1 bis 3
**dadurch gekennzeichnet,**
**daß** der Innenraum der der Lüftungsbohrung (6) zugeordneten Kammer (12; 13) gleich lang oder kürzer als der der Be- und Entladeöffnung (5) zugeordneten Kammer (14) ist.

5. Adapterstopfen gemäß einem der Ansprüche 1 bis 4
**dadurch gekennzeichnet,**
**daß** an der Außenseite der Steckhülse (4) ein oder mehrere radial umlaufende Lippen (20) angebracht sind.

## Claims

1. An adapter plug for a receptacle for bodily fluids which includes a tapered mounting member (3) for receiving a tapered needle (7) of a syringe (1) and a vent bore (6), which communicates with an insertion sleeve (4) on the receptacle side of the adapter plug, wherein mounted in the insertion sleeve (4) there is a partition (10; 11), which divides the interior of the insertion sleeve into a vent chamber (12; 13) and an injection chamber (14) and that the supply and discharge opening (5) communicates with the injection chamber (14) and the vent bore (6) communicates with the vent chamber (12; 13), **characterised in that** the vent bore is so positioned in a rear region of the insertion sleeve that, when the insertion sleeve is fully inserted into the receptacle, it is sealed by the inner wall of the receptacle.

2. An adapter plug as claimed in Claim 1, **characterised in that** the spacing of the vent bore (6) from the free end of the insertion sleeve (4) is greater than that of the front end face of the partition (11) from the free end of the insertion sleeve (4).

3. An adapter plug as claimed in Claim 1 or 2, **characterised in that** the partition (10) extends over the entire internal length of the insertion sleeve (4).

4. An adapter plug as claimed in one of Claims 1 to 3, **characterised in that** the interior of the chamber (12; 13) associated with the vent bore (6) is of the same length or shorter than the chamber (14) associated with the supply and discharge opening (5).

5. An adapter plug as claimed in one of Claims 1 to 4, **characterised in that** one or more radially circular lips (20) are attached to the outer surface of the insertion sleeve (4).

## Revendications

1. Bouchon adaptateur pour un récipient de réception de liquides corporels comprenant un cône à enficher (3) pour la réception d'un cône aiguille (7) d'une seringue (1), ainsi qu'un passage de ventilation (6) débouchant dans un manchon d'enfichage (4), du côté du récipient de réception, du bouchon adaptateur, dans lequel
est disposée dans paroi de séparation (10 ; 11) qui divise l'espace intérieur du manchon d'enfichage en une chambre de ventilation (12 ; 13) et en une chambre d'injection (4), et l'ouverture (5) de chargement et de chargement débouche dans la chambre d'injection (14), et l'ouverture de ventilation (6) débouche dans la chambre de ventilation (12 ; 13), **caractérisé en ce que** le passage de ventilation est disposé dans une zone arrière du manchon d'enfichage, de sorte qu'il est fermé par la paroi interne du récipient de réception lorsque le manchon d'enfichage est entièrement enfiché dans le récipient de réception.

2. Bouchon adaptateur selon la revendication 1,
**caractérisé en ce que**,
la distance comprise entre l'ouverture de ventilation (6) et l'extrémité libre du manchon d'enfichage (4) est supérieure à celle comprise entre la face frontale avant de la paroi de séparation (11) et l'extrémité libre du manchon d'enfichage (4).

3. Bouchon adaptateur selon la revendication 1 ou 2,
**caractérisé en ce que**,
la paroi de séparation (10) s'étend sur toute la longueur interne du manchon d'enfichage (4).

4. Bouchon adaptateur selon l'une des revendications 1 à 3,
**caractérisé en ce que**,
l'espace intérieur de la chambre (12 ; 13) associée à l'ouverture de ventilation (6) est de même longueur ou plus court que la chambre (14) associé à l'ouverture (5) de chargement et déchargement.

5. Bouchon adaptateur selon l'une des revendications 1 à 4,
**caractérisé en ce que**,
une ou plusieurs lèvres (20) s'étendant radialement est/sont placée(s) sur la face externe du manchon d'enfichage (4).
